Europäisches Patentamt

European Patent Office

Office européen des brevets

(11) Publication number: **0 229 042**
A2

## (12) EUROPEAN PATENT APPLICATION

(21) Application number: 87420005.8

(22) Date of filing: 07.01.87

(51) Int. Cl.⁴: **C 12 N 15/00**
C 12 N 1/20, C 12 N 5/00
C 12 N 9/78

(30) Priority: 08.01.86 US 817226
28.03.86 US 845662

(43) Date of publication of application:
15.07.87 Bulletin 87/29

(84) Designated Contracting States:
ES GR

(71) Applicant: RHONE-POULENC AGROCHIMIE
14-20, rue Pierre Baizet
F-69009 Lyon(FR)

(72) Inventor: Stalker, David
3317 Oyster Bay Avenue
Davis California 95616(US)

(74) Representative: Chrétien, François et al,
RHONE-POULENC AGROCHIMIE BP 9163
F-69263 Lyon Cedex 09(FR)

(54) Haloarylnitrile degrading gene, its use, and cells containing the gene.

(57) Nitrilase enzymes specific for the hydrolysis of the nitrile group of benzonitriles, eg. bromoxynil or ioxynil, nucleotide sequences encoding for such enzymes, and transformed cells in which the nitrilase expression is foreign are provided. The transformed cells are capable of expressing the nitrilase enzyme to provide detoxification of an environment and protect benzonitrile herbicide-sensitive cells from its cytotoxic effect. Plants are developed which are resistant to bromoxynil.

EP 0 229 042 A2

The present invention relates to a haloarylnitrile degrading gene, its use, cells containing the gene and nitrilase enzyme obtainable therefrom.

The opportunity to provide novel genetic capabilities to microorganisms and cells of higher organisms has opened up broad avenues to new capabilities. In one arena is the concern with various agents that are utilized for their cytotoxic effect. For example, many compounds used in agriculture are directed to the killing of pests, or weeds. In many cases, these compounds can have a relatively long residence time or extended residue.

In many situations, one wishes to distinguish between species which are to be retained and species which are to be killed. For example, it is frequently desirable to selectively kill weeds while having minimal adverse effect on crops. For the most part, many of the broad spectrum herbicides have a significant adverse effect on the crop, so that their use is primarily limited to preemergent use or careful postemergent application.

It is therefore of great interest to be able to modify viable cells to make them resistant to stresses caused for example by cytotoxic agents. US Patent No. 4,535,060 describes the use of a bacterial aroA gene to impart glyphosate resistance to glyphosate susceptible cells.

Hsu and Camper, Can. J. Microbiol (1976) 22:537-543, describe the isolation of ioxynil degraders from soil-enrichment cultures. Hsu and Clemson, Dissert. Abstr. Intrn. B36 (1976) No. 8, 3708, describe microbial degradation of a family of herbicides of 3,5-dihalogeno-4-hydroxybenzo-nitriles. Ingram and Pullin, Pestic. Sci. (1974) 5:287-291 describe the persistence of bromoxynil in three soil types. Smith, Abstrp. Meeting Weed Soc. Am. (1971), pp. 16-17 describes the degradation of bromoxynil in Regina heavy clay. Smith and Fletcher, Hort. Res. (1964), 4:60-62, report on 3,5-dihalogeno-4-hydroxybenzonitriles and soil microorganisms.

The present invention relates to nitrilases, nucleic acid sequences encoding such nitrilases, constructs containing the genes coding such nitrilases under the transcriptional and translational regulatory control of regulatory genes recognised by a desired host to which the nitrilase genes are foreign, host cells containing such constructs, and organisms and organism parts or products containing such constructs. In particular the bromoxynil- and/or ioxynil-specific nitrilases find use for detoxifying habitats containing these and related herbicides and protecting host cells from the cytotoxic effect of such herbicides. The constructs find use in distinguishing between host cells containing the construct and host cells lacking such construct.

In accordance with the present invention, novel DNA sequences, constructs, transformed cells, plants, and peptides are provided relating to the hydrolysis of halogenated hydroxybenzonitriles, particularly 3,5-dibromo- or 3,5-diiodo-4-hydroxybenzonitrile (which are known as bromoxynil and ioxynil, respectively). The invention concerns the production of an enzyme capable of hydrolysing the nitrile so as to detoxify the herbicidal activity of the nitrile and provide protection to a cell or host sensitive to the herbicide or detoxify an environment contaminated with the herbicide.

The structural gene of interest may be obtained from a unicellular microorganism, particularly a bacterium, which is shown to be capable of employing the benzonitrile as a nitrogen source, usually being capable of employing the benzonitrile as the exclusive nitrogen source. Hereinafter, in referring to a benzonitrile or a nitrilase, it is to be understood that the benzonitrile is a halogenated p-hydroxybenzonitrile, preferably 3,5-diiodo- or 3,5-dibromo-4-hydroxybenzonitrile, and the nitrilase is a nitrilase which is capable of using such a benzonitrile as a nitrogen source, particularly as its exclusive nitrogen source.

The enzyme can be obtained in different ways, conveniently from bacteria which exist naturally im an environment containing, for example, bromoxynil or ioxynil. Particularly, enteric bacteria, more particularly of the species Klebsiella, are of interest. Klebsiella pneumoniae may be employed, more particularly var. ozaenae. Instead of isolation from soil, organisms may be grown in soil or other medium at increasingly higher concentrations of the benzonitrile and reduced amounts of alternative nitrogen sources until organisms which survive employing the benzonitrile as the sole nitrogen source are obtained.

Regardless of the source of the bacterium containing the nitrilase, screening must be performed to ensure that the nitrilase is efficient in the detoxification of the benzonitrile. In addition, the nitrilase should be specific for the benzonitrile rather than other analogues, which lack the halogens or which have other substituents. The nitrilases of this invention will therefore be specific for the benzonitriles  as defined, and be relatively inactive toward  analogues or substantially less active toward analogues. Desirably, there should be no significant reduction in rate of proliferation, that is, less than about 10% reduction, of the proliferation of the bacterium in the presence of a normal nitrogen source, e.g. ammonia, as compared to the benzonitrile as the nitrogen source at comparable concentrations. Such result will not be observed with non-specified benzonitriles.

Once one or more host strains have been identified, techniques may then be employed to identify the coding sequence for the nitrilase. The gene may be present on a chromosome or plasmid. The genome may be fragmented, particularly with a restriction endonuclease, where one or a multiple of endonucleases may be employed to provide fragments ranging from about 5kb to 50kb. These fragments may be cloned on appropriate vectors in a convenient bacterium, e.g., E. coli, and the resulting transformants screened for nitrilase activity, where the host organism provides a negative background.

Once one or more clones have been identified as having nitrilase activity, the extrachromosomal elements containing the desired DNA fragment, plasmids or viruses, may be isolated by conventional techniques, such as lysis of the host, precipitation of the DNA, and separation of the vector DNA, plasmid or virus DNA, from the chromosomal DNA. The extrachromosomal elements may then be cleaved by endonuclease restriction and the desired fragments isolated by various techniques for separation and identification of different sized fragments, e.g., electrophoresis or density gradient centrifugation.

Depending upon the size of the fragment, it will usually be further manipulated to reduce the size to more closely approximate the size of the gene and its flanking regulatory regions. Various techniques exist for manipulating the fragment containing the sequence coding for the enzyme and its regulatory flanking sequences. Partial cleavage with different restriction enzymes in different reaction mixtures may be employed, followed by cloning of the fragments to determine which fragments still retain the ability to provide expression of the nitrilase.

Alternatively, the enzyme may be isolated and partially sequenced. Based on the amino acid sequence, probes can be prepared which may then be used to identify those fragments having the gene. By combining this approach with restriction enzyme cleavage, fragments can be cloned and screened for the presence of the desired gene. In addition, one may use exo- nucleases, such as Bal31 to remove nucleotides from one or both ends of the fragment to further reduce the number of superfluous nucleotides.

Alternatively, the gene may be cloned in an appropriate host and messenger RNA isolated by screening with a probe, by identification in an appropriate in vitro or in vivo translation system, e.g., Xenopus oocytes or reticulolysate. The isolated messenger may then be used for preparing cDNA using conventional techniques involving a reverse transcriptase and formation of the complementary chain with a DNA polymerase. In this instance, the resulting structural gene lacks the regulatory regions associated with transcription.

The DNA sequence containing the structural gene expressing the nitrilase may be joined to a wide variety of other DNA sequences for introduction into an appropriate host cell. The companion sequence will depend upon the nature of the host, the manner of introduction of the DNA sequence into the host, and whether episomal maintenance or integration is desired.

For prokaryotic hosts, a wide variety of vectors exist which may be used for introduction by transformation, conjugation, transduction or transfection of the DNA sequence into a prokaryotic host. Vectors capable of introducing DNA sequences include a wide variety of plasmids, such as pBR322, pACYC184, pMB9, pRK290; cosmids, such as pVK100; or a virus, such as P22.

For eukaryotic hosts, a wide variety of techniques may be employed for DNA introduction into the host, such as transformation with $Ca^{++}$-precipitated DNA, involving a non-replicating DNA sequence, a plasmid or a minichromosome, transformation with a T-DNA containing sequence in Agrobacterium, microinjection with a micropipette, or electroporation. Depending upon whether a competent replication system is present in the DNA construction, will determine whether the DNA may be replicated as an episomal element, or the DNA may be integrated into the host genome, and the structural gene expressed in the host. Episomal elements may be employed, such as tumor inducing plasmids, e.g., Ti or Ri, or fragments thereof, or viruses, e.g., CaMV, TMV or fragments thereof, which are not lethal to the host, and where the structural gene is present in such episomal elements in a manner allowing for expression of the structural gene. Particularly of interest are fragments having the replication function and lacking other functions such as oncogenesis or virulence.

The fragments obtained from the nitrilase source may be cloned employing an appropriate cloning vector. Cloning can be carried out in an appropriate unicellular microorganism, e.g., a bacterium, such as E. coli. Desirably, one may use a cosmid, where partial or complete digestion provides fragments having about the desired size. For example, the cosmid pVK100 may be partially digested with an appropriate restriction enzyme and ligated to fragments resulting from either partial or complete digestion of a plasmid, chromosome, or fragment thereof. Packaging will insure that only fragments of the desired size will be packaged and transduced into the host organism.

The host organism may be selected for benzonitrile resistance. The recipient strains may be modified to provide for appropriate genetic traits which allow for selection of transductants. In microorganisms, the transductants may be used for conjugation to other microorganisms, using a mobilizing plasmid as required. Various techniques may be used for further reducing the size of the fragment containing the structural gene for the nitrilase. For example, the cosmid vector may be isolated, cleaved with a variety of restriction endonucleases, e.g., EcoRI, BglII, SmaI, and the resulting fragments cloned in an appropriate vector, conveniently the cosmid vector previously used. Instead of a cosmid vector, a variety of cloning vectors are available of small size, such as pACYC177 and pACYC184. Thus, fragments of preferably less than about 5kb, usually less than about 4kb, and more preferably less than about 2kb, can be cloned and provide for benzonitrile resistance.

Desirably, the fragment will be about 1kb and less than about 5kb, preferably less than about 4kb, particularly at least about 1047bp, more particularly including flanking regions of at least about 1100bp, preferably less than about 1.5kb. Of particular interest, is a BglII-SmaI fragment from Klebsiella ozaenae, more particularly a PstI-HincII fragment of about 1210bp.

The invention provides a DNA sequence encoding a 3,5-dihalogenated-p-hydroxybenzonitrile specific bacterial nitrilase and which is joined to and under the transcriptional regulatory region control of other than the wild type transcriptional initiation region for a bromoxynil and/or ioxynil specific bacterial nitrilase found in Klebsiella. The transcriptional regulatory region may be, for example, functional in a plant or in a bacterium.

The invention further provides a DNA sequence having an open reading frame coding for a nitrilase enzyme specific for a 3,5-dihalogenated-p-hydroxybenzonitrile, the sequence having at its 5'-terminus other than the wild type transcriptional initiation region. The nitrilase enzyme is preferably a bacterial nitrilase enzyme. The DNA sequence is preferably substantially homologous with a DNA sequence from Klebsiella.

The nitrilase enzymes may be expressed by any convenient source, either prokaryotic or eukaryotic, including bacteria, yeast, filamentous fungus and plant cells. Where secretion is not obtained, the enzyme may be isolated by lysing the cells and isolating the nitrilase according to known ways. Useful ways include chromatography, electrophoresis and affinity chromatography. Conveniently, the benzonitrile, eg.

bromoxynil or ioxynil may be conjugated through an appropriate functionality, e.g. the carboxyl group, to an insoluble support and used as a packing for the isolation of the nitrilase.

The invention provides a composition comprising a nitrilase (preferably a bacterial nitrilase, most preferably from Klebsiella) having a molecular weight of about 34kDal, the composition having a specific activity of at least about 0.1 $\mu$mol $NH_3$/min/mg protein with bromoxynil as substrate.

The invention also provides a process for the preparation of a 3,5-dihalogenated-p-hydroxybenzonitrile specific nitrilase which comprises culturing a bacterial host according the invention.

The nitrilase specific activity will be at least about 0.1 $\mu$mol ammonia/min/mg protein, generally at least about 0.5 or higher under conditions as described by Harper, Biochem. J. (1977) 167:685-692.

The purified enzyme can be used in a wide variety of ways. It may be used directly in assays for bromoxynil, ioxynil, or other related benzonitriles. Alternatively, the subject enzyme can find use as a label in diagnostic assays, by being conjugated to an analyte of interest, e.g. a hapten or antigen, or to an antibody, as such assays are described in U.S. Patent Nos. 3,654,090; 3,817,837; and 3,850,752. The methods of conjugation, as well as the determination of the concentration of an analyte are described in extensive detail in these patents.

The DNA sequence encoding for the nitrilase may be used in a variety of ways. The DNA sequence may be used as a probe for isolation of wild type or mutated nitrilases. Alternatively, the DNA sequence may be used for integration by recombination into a host to provide for imparting benzonitrile resistance to the host.

With plant cells, the structural gene as part of a construction may be introduced into a plant cell nucleus by micropipette injection for integration by recombination into the host genome. Alternatively, electroporation may be employed into which the structural gene may be introduced for introduction into a plant host. Where the structural gene has been obtained from a source having regulatory signals which are not recognized by the plant host, it may be necessary to introduce the appropriate regulatory signals for expression. Where a virus or plasmid, e.g. tumor inducing plasmid, is employed and has been mapped, a restriction site can be chosen which is downstream from a promoter into which the structural gene may be inserted at the appropriate distance from the promoter. Where the DNA sequences do not provide an appropriate restriction site, one can digest for various times with an exonuclease, such as Bal31 and insert a synthetic restriction endonuclease site (linker).

Of particular interest is the use of a tumor-inducing plasmid, e.g., Ti or Ri, where the nitrilase gene may be integrated into plant cell chromosomes. Descriptions of the use of Ti-plasmids and Ri-plasmids may be found in PCT Publication Nos. WO84/02913, 02919 and 02920 and EPO Application 0 116 718, and Matzke and Chilton, J. Mol. App. Genetics (1981) 1:39-49.

By employing the T-DNA right border, or both borders, where the borders flank an expression cassette comprising the nitrilase structural gene under transcriptional and translational regulatory signals for initiation and termination recognized by the plant host, the expression cassette may be integrated into the plant genome and provide for expression of the nitrilase enzyme in the plant cell at various stages of differentiation.

Various constructs can be prepared providing for expression in plant cells. The constructs provide an expression cassette which is functional in plants for expression of the nitrilase in the plant host.

To provide for transcription, a variety of transcriptional initiation regions (promoter regions), either constitutive or inducible, may be employed.

The transcriptional initiation region is joined to the structural gene encoding the nitrilase to provide for transcriptional initiation upstream from the initiation codon, normally within about 200 bases of the initiation codon, where the untranslated 5'-region lacks an ATG.

The 3'-end of the structural gene will have one or more stop codons which will be joined to a transcriptional termination region functional in a plant host, which termination region may be associated with the same or different structural gene as the initiation region.

The expression cassette is characterized by having in the direction of transcription the initiation region, the structural gene under the transcriptional control of the initiation region, and the termination region providing for termination of transcription and processing of the messenger RNA, as appropriate.

As transcriptional and translational regulatory regions, conveniently opine promoter and terminator regions may be employed, which allow for constitutive expression of the nitrilase gene. Alternatively, other promoters and/or terminators may be employed, particularly promoters which provide for inducible expression or regulated expression in a plant host. Promoter regions which may be used from the Ti-plasmid include opine promoters, such as the octopine synthase promoter, nopaline synthase promoter, agropine synthase promoter, and manopine synthase promoter. Other promoters include viral promoters, such as CaMV Region VI promoter or full length (35S) promoter, the promoters associated with the ribulose-1,5-bisphosphate carboxylate genes, e.g. the small subunit, genes associated with phaseolin, protein storage, $\beta$-conglycinin, and cellulose formation.

The various sequences may be joined together in conventional ways. The promoter region may be identified by the region being 5' from the structural gene, for example, the opine gene, and by restriction mapping and sequencing may be selected and isolated. Similarly, the terminator region may be isolated as the region 3' from the structural gene. The sequences may be cloned and joined in the proper orientation to provide for constitutive expression of the nitrilase gene in a plant host.

The present invention accordingly provides an expression cassette comprising a structural gene coding for a 3,5-dihalogenated-p-hydroxybenzonitrile specific nitrilase under the transcriptional and translational regulatory control of regulatory regions functional in a plant cell. The nitrilase is preferably a bacterial nitrilase. The cassette preferably has at least one T-DNA border. The transcriptional initiation region in the cassette is preferably from a gene encoding an opine.

The invention further provides a vector, eg. a plasmid, which incorporates an expression cassette according to the invention. Plasmids according to the invention are preferably capable of replication in E. coli or A. tumefaciens.

The invention also provides a bacterial host (preferably E. coli) having a foreign gene capable of expressing a nitrilase specific for a 3,5-dihalogenated-p-hydroxybenzonitrile.

By modifiying crop plant cells by introduction of a functional gene expressing the nitrilase enzyme, one can use bromoxynil, ioxynil, or analogous benzonitrile herbicide with a wide variety of crops at concentrations which ensure the substantially complete or complete removal of weeds, while leaving the crop relatively unaffected. In this manner, substantial economies can be achieved in that fertilizers and water may be more efficiently utilized, and the detrimental effects resulting from the presence of weeds avoided.

The expression cassette expressing the nitrilase enzyme may be introduced into a wide variety of plants, both monocotyledon and dicotyledon, including maize, wheat, soybean, tobacco, cotton, tomatoes, potatoes, Brassica species, rice, peanuts, petunia, sunflower, sugar beet and turfgrass. The gene may be present in cells or plant parts including callus, tissue, roots, tubers, propagules, plantlets, seed, leaves, seedlings and pollen.

By providing for benzonitrile-resistant plants, a wide variety of formulations may be employed for protecting crops from weeds, so as to enhance crop growth and reduce competition for nutrients. For example, bromoxynil could be used by itself for post-emergence control of weeds with bromoxynil-resistant crops, such as sunflower, soybeans, corn or cotton or alternatively, in combination formulations with other products. The invention provides a plant cell comprising an expression cassette according to the invention and further provides a plant comprising such a cell.

Conventional amounts of the herbicides would be applied to fields in the formulations to deliver from about 0.1 to 4 lb/acre, preferably 0.2 to 2 lb/acre, of benzonitrile such as bromoxynil or ioxynil, where the other herbicide would be in amounts to deliver from about 0.1 to 4 lb/acre of active ingredient. Formulations would include other additives, such as detergents, adjuvants, spreading agents, sticking agents or stabilizing agents. The formulations may either be wet or dry formulations, including flowable powders, emulsifiable concentrates and liquid concentrates, as is known in the art.

The herbicidal solutions may be applied in accordance with conventional ways, for example, by spraying, irrigation or dusting.

The following Example illustrates the invention. E. coli MM294 strain (pBrx5), referred to in the following Example was deposited at the A.T.C.C. on January 22, 1986, and given Accession No. 53435. The strain, and a culture thereof in a medium comprising a source of assimilable carbon, a source of assimilable nitrogen and optionally, mineral elements and substantially free of other microorganisms, constitute features of the invention.

<div align="center">EXAMPLE</div>

## Materials and Methods

Restriction enzymes and T4 ligase for ligations were utilized according to the manufacturer's recommendations. Standard methods in cloning and molecular analysis were performed according to Maniatis et al., (1982) Molecular Cloning: A Laboratory Manual, Cold Spring Harbor Laboratory, New York. Clone analysis was performed as described by Ish-Horowitz et al., Nucl. Acids Res. (1981) 9:2989-2998.

E. coli strain MM294 was used for all cloning experiments. (Hanahan, Mol. Biol. (1983) 166:557-80.)

The levels of antibiotics when employed were: Cm (chloramphenicol) 25 μg/ml; Tc (tetracycline) 10 μg/ml; Ap (penicillin) 300 μg/ml.

Transformations of plasmid DNAs in $\underline{E.}$ $\underline{coli}$ were performed according to Mandel and Higa, $\underline{J.\ Mol.}$ $\underline{Biol.}$ (1970) $\underline{53}$:159-162.

Bacterial isolates from a bromoxynil contaminated soil sample were isolated and screened. One such organism was identified as $\underline{Klebsiella}$ $\underline{pneumoniae}$ sub-species $\underline{ozaenae}$. Partial purification and characterization of the bromoxynil specific nitrilase from the above organism yielded an active enzyme with an apparent molecular weight of 34kDal.

Upon repeated subculturing of $\underline{K.}$ $\underline{ozaenae}$ on solid L- agar, a variant was isolated which no longer was able to utilize bromoxynil as a sole nitrogen source when this variant organism was grown in defined liquid medium containing per liter $KH_2PO_4$ (1.5g), $K_2HPO_4$ (3.5g), $MgSO_4 \cdot 7H_2O$ (0.1g), yeast extract (50mg), citrate, glycerol and succinate at 0.1%, and trace elements as described by Barnett and Ingraham, $\underline{J.\ Appl.}$ $\underline{Bacteriol.}$ (1975) 18:131-143. This medium henceforth will be known as YETE multi-carbon medium. The YETE multicarbon medium contained 0.05% bromoxynil. Although this organism did not utilize bromoxynil as sole nitrogen source, it would grow to full density in L-broth containing 0.05% bromoxynil. A $\underline{K.}$ $\underline{ozaenae}$ variant colony was selected and grown in 10mls of L-broth. Three independent $\underline{K.}$ $\underline{ozaenae}$ colonies were also chosen from a LB plate containing bromoxynil and grown under the same conditions. These same four $\underline{K.}$ $\underline{ozaenae}$ colonies were simultaneously grown in 10mls L-broth supplemented with 0.05% bromoxynil. Cultures were grown to full density to 30°C and mini-prep plasmid DNA prepared from each culture by the method of Ish-Horowitz $\underline{et}$ $\underline{al.}$, $\underline{Nucl.\ Acids\ Res.}$ (1981) $\underline{9}$:2989. Undigested plasmid DNAs were electrophoresed on a 0.5% agarose gel and the plasmid bands visualized by ethidium bromide staining.

The K. ozaenae variant organism revealed a single plasmid species (68Kb in size) grown either in the presence or absence of bromoxynil. The three K. ozaenae colonies showed a larger plasmid species (90Kb) when grown in the presence of 0.05% bromoxynil. In the absence of bromoxynil, both plasmid forms are present in two of the three K. ozaenae colonies. This data indicates conversion of the larger plasmid species to a smaller form with the concommitant loss of approximately 22Kb of plasmid DNA when bromoxynil selection is relieved.

All four colonies were grown in 200mls L-broth containing 0.05% bromoxynil. Cells were disrupted with a French press, the high speed supernatants dialyzed against buffer containing 0.05M $KPO_4$ pH7.5; 2.5mM dithiothreitol (DTT) and the individual crude extracts assayed for bromoxynil specific nitrilase activity. A crude extract prepared from the K. ozaenae variant contained no detectable nitrilase activity while the other K. ozaenae crude extracts exhibited nitrilase specific activities of 0.124, 0.105 and 0.143μmole $NH_3$/min/mg protein respectively. Cells (200ml) were grown at 30°C to mid log phase in M9 medium (Miller (1972) Experiments in Molecular Genetics, Cold Spring Harbor Laboratory) containing 0.1% glucose and 0.04% bromoxynil. Crude extracts were prepared by cell disruption, ultracentrifugation and dialysis of the supernatant in buffer containing 0.05M $KPO_4$ pH 7.5 and 2.5mM DTT. Substrate concentration was 3mM bromoxynil in all assays. Release of $NH_3$ was monitored according to Harper Biochem. J. (1977) 167:685-692. The ability of K. ozaenae variant to grow in L-broth containing bromoxynil may result in acquired impermeability of the organism to the compound. However, the organism cannot grow in defined media utilizing bromoxynil as sole nitrogen source.

In summary, the K. ozaenae nitrilase appears to be plasmid encoded. The gene(s) encoding the enzyme appears to reside on a 22Kb plasmid DNA segment spontaneously lost from the K. ozaenae plasmid in the absence of bromoxynil selection.

The K. ozaenae bromoxynil specific nitrilase
is expressed in E. coli.

Plasmid DNA from K. ozaenae grown under 0.05% bromoxynil selection was prepared and the DNA transformed to E. coli strain MM294 (thi, gyrA96, endI⁻, hsdR17). Transformants were selected on nitrogen deficient (N⁻) solid agarose minimal medium (containing per liter $KH_2PO_4$ (1.5g), $K_2HPO_4$ (3.5g), $MgSO_4 \cdot 7H_2O$ (0.1g) and 0.1% glucose) with the addition of 0.05% bromoxynil as sole nitrogen source. After 5 days incubation, 10 colonies appeared on the selective plates. These colonies were restreaked on L-agar plates containing 0.05% bromoxynil and tested for the presence of the thiamine auxotrophic marker in MM294. None of the colonies grew in minimal media in the absence of thiamine indicating the strain to be E. coli MM294. All colonies could grow in M9 medium supplemented with thiamine and 0.05% bromoxynil as sole nitrogen source. No growth was observed in this medium in the absence of bromoxynil. Two of the colonies were selected for further analysis. When crude extract preparations of E. coli MM294 containing the 90kb plasmid were assayed for bromoxynil specific nitrilase activity, a specific activity of $0.216 \mu$mole $NH_3$ released/min/mg was obtained. E. coli MM294 containing the smaller plasmid species produced no detectable nitrilase activity. The larger 90Kb plasmid in E. coli was designated pBrx1 while the smaller plasmid (68Kb) was designated pBrx1Δ.

To confirm that E. coli strain MM294 containing plasmid pBrx1 produces the proper metabolite as a result of a bromoxynil specific nitrilase reaction, a 2ml culture of MM294 (pBrxl) was grown for 24hr at 30°C in M9 medium supplemented with 0.05% bromoxynil. A culture filtrate sample was chromatographed on a $C_{18}$ HPLC column. All input bromoxynil in the culture filtrate was converted to a new metabolite peak. The identity of the metabolite peak was determined by spectral analysis to be 3,5-dibromo-4-hydroxybenzoic acid (DBHB). Thus, the product of the bromoxynil specific plasmid encoded nitrilase expression in E. coli is the same as that observed for K. ozaenae.

The bromoxynil specific nitrilase gene is cloned in E. coli.

To determine whether the DNA segment encoding the bromoxynil specific enzyme is clonable in E. coli, plasmid pBrxl was digested with BamHI resulting in two bands of 53Kb and 37Kb, respectively. The BamHI fragments were ligated into the BamHI site of the E. coli plasmid vector pACYC184 (Chang and Cohen, J. Bacteriol. (1978) 134:1141) and transformed to E. coli strain MM294. Cloning into the BamHI site of pACYC184 results in insertional inactivation of the tetracycline resistance gene. Ten chloramphenicol resistant tetracycline sensitive MM294 colonies were selected, mini-prep clone analysis DNA prepared and the DNA digested with BamHI. Four clones contained the 37Kb BamHI fragment while one clone harbored the larger 53Kb BamHI DNA fragment of pBrxl. Five clones contained a cloned BamHI fragment also found in plasmid pBrxlΔ which corresponds to the DNA segment remaining after spontaneous deletion of 22Kb of plasmid DNA from pBrxl. All 10 clones were grown in 200ml L-broth in the presence of 20µg/ml chloramphenicol (to select for the plasmid), crude extract preparations obtained and assayed for bromoxynil specific nitrilase activity. Four clones containing the 37Kb BamHI fragment exhibited nitrilase specific activities in the range of 0.140μmole $NH_3$ released/min/mg protein while no detectable nitrilase activity was observed in the other six clones. This data indicates the gene encoding a bromoxynil specific nitrilase activity is located on a 37Kb BamHI fragment cloned from plasmid pBrx1 and that the 22Kb DNA segment spontaneously lost in the absence of bromoxynil selection is internal to the 37Kb BamHI fragment.

To confirm the orientation of the BamHI fragments with respect to the vector pACYC184, DNA from the above four clones was digested with EcoRI and electrophoresed on a 0.07% agarose gel. A combined EcoRI digest of plasmids pBrx1 and pBrx1Δ was also analyzed.

Both orientations of the 37Kb BamHI fragment with respect to the vector pACYC184 were defined and designated plasmids pBrx2 and pBrx3, respectively. It was also observed that the three EcoRI fragments are internal to the 22Kb DNA segment that is spontaneously deleted from plasmids pBrx2 and pBrx3. The sizes of these EcoRI fragments are 18Kb, 3Kb and 1.9Kb, respectively. The gene encoding the bromoxynil specific nitrilase should be located within one of these three EcoRI fragments if the nitrilase structural gene is not bisected by an EcoRI restriction site.

Localization of the bromoxynil specific nitrilase of E. coli (pBrx3) was investigated. The results were as follows.

| TABLE 1 |
| --- |

The Bromoxynil Specific Nitrilase is a
Periplasmic Enzyme in E. coli.

| Culture Conditions[a] | Nitrilase Specific Activity[b] |
| --- | --- |
| toluenized cells (L-broth) | 0.829 |
| lysozyme treated cells (L-broth) | 0.796 |
| whole cells (L-broth) | 0.770 |
| whole cells (L-broth + Brxl) | 1.25 |
| whole cells (M9) | 0.950 |
| whole cells (M9 + Brxl) | 1.45 |
| whole cells/pACYC184 (M9) | 0 |

[a] E. coli (MM294) cells containing plasmid pBrx3 were grown to stationary phase in 5ml cultures at 37° in medium indicated. Cultures contained 20µg/ml chloramphenicol and 0.04% bromoxynil (Brxl) where indicated. One ml from each culture was harvested, washed once with nitrilase buffer (0.1M $KPO_4$ pH7.5) and cells resuspended in 0.1ml of this same buffer. 50µl samples were assayed for nitrilase activity according to Harper, Biochem. J. (1977) 167:685-692, with and without 3mM bromoxynil as substrate.

[b] µmole $NH_3$/min/mg. Protein was determined as $O.D._{600}$ of 1.4 - $10^9$ cells/ml = 150µg.

These data indicate that the cellular location of the nitrilase enzyme is the periplasmic space. A second observation is that the enzyme is expressed in the absence of bromoxynil in the medium suggesting that bromoxynil induction is not required for enzyme expression.

<u>Further purification of the bromoxynil specific</u>
<u>nitrilase.</u>

Further purification of <u>K</u>. <u>ozaenae</u> nitrilase was carried out with the following results.

---

## TABLE 2

Purification from <u>E</u>. <u>coli</u> of the Bromoxynil Specific Nitrilase.
(Starting material 6gms cells)

| <u>Fraction</u> | <u>Volume</u> | <u>Protein</u> | <u>μmole NH$_3$/min</u> | <u>S.A.</u>[b] |
|---|---|---|---|---|
| Crude[a] | 100ml | 210mg | 18.15 | 0.086 |
| 35-50%<br>NH$_4$SO$_4$ | 6ml | 83mg | 26.77 | 0.250 |
| DEAE<br>Sephadex | 56ml | 19mg | 15.52 | 0.820 |

---

[a]  Cells were grown at 30° to mid log phase in M9 medium containing 0.04% bromoxynil and glucose. Crude extracts were prepared by cell disruption, ultracentrifugation and dialysis in buffer containing 0.05M KPO$_4$ pH7.5 and 2.5mM DTT. Substrate concentration was 3mM in all nitrilase assays.

[b]  μmole NH$_3$/min /mg

A 2.5cm$^2$ x 10cm column was equilibrated in buffer containing 0.05M KPO$_4$ pH7.5, 2.5mM DTT and 1mM EDTA. The sample was applied and the column developed with a 300ml linear gradient of 0.02M to 0.40M NaCl in the above column buffer. Buffer containing 1M NaCl was applied at the end of the gradient. 5ml fractions were collected and 0.075ml aliquots of alternate fractions assayed for nitrilase activity. A single peak of enzyme activity eluted at 0.22M salt. Approximately 75% of the input nitrilase activity was recovered in the the active fractions.

Fractions spanning the nitrilase peak from the DEAE column were dialyzed against 0.02M KPO$_4$ pH7.5 and 50μl (6μg protein) of each fraction applied to an 11.25% denaturing Laemmli gel. The enriched protein band that corresponds to the activity peak from the DEAE column is a polypeptide of 34,000 Daltons molecular weight. No other polypeptides were enriched by the active column fractions. These data indicate that the bromoxynil specific nitrilase is a polypeptide of approximately 34,000 Daltons molecular weight and probably the product of a single gene.

Clone pBrx2 was completely digested with EcoRI and an approximately 19kb fragment isolated. The fragment was inserted into the EcoRI-digested pACYC184 vector (3.9kb) to provide the plasmid pBrx5 which was transformed into E. coli as described previously. The plasmid was isolated in conventional ways and digested with BglII to provide an approximately 6.7kb fragment which remained inserted in the pACYC184 vector. The isolated plasmid pBrx7 was then digested with SmaI and BglII to provide an approximately 3.9kb fragment which was inserted into SmaI-BamHI digested pACYC177 (3.7kb) (Chang and Cohen, J. Bacteriol. (1978) 134:1141-1156). The resulting plasmid which provided penicillin resistance was transformed into E. coli as described previously and transformants selected on penicillin selected medium to provide plasmid pBrx8, which carries the nitrilase gene on a 3.9kb fragment.

pBrx8 is partially digested with PstI and the fragments inserted into PstI digested pUC18 (Yanisch-Perron et al., Gene (1985) 33:103-119). The resulting plasmids were cloned in E. coli and screened for nitrilase activity. One clone had a 5.3kb plasmid pBrx9 which was isolated and further digested with PstI and HincII resulting in a 1210bp fragment having in the direction of PstI to HincII, ClaI, SalI; ScaI, and SphI restriction sites relatively evenly spaced. The PstI-HincII fragment was sequenced according to the method of Sanger et al., Proc. Natl. Acad. Sci. USA (1977) 74:5463-5468. The resulting sequence (with the appropriate amino acids encoded) is set forth in the following sequence.

CTGCAGGATAGTAGGGGCTTGAAGAGGATACGCTGTTTGGCGAGCCATCAAAATAAGGGGATTTTC

```
                                      95                                    125
ATG GAC ACC ACT TTC AAA GCA GCC GCT GTT CAG GCC GAA CCG GTA TGG ATG GAT GCC GCT
Met Asp Thr Thr Phe Lys Ala Ala Ala Val Gln Ala Glu Pro Val Trp Met Asp Ala Ala

                                     155                                    185
GCA ACA GCC GAT AAG ACC GTG ACG CTA GTA GCT AAA GCC GCA GCG GCT GGC GCG CAG CTC
Ala Thr Ala Asp Lys Thr Val Thr Leu Val Ala Lys Ala Ala Ala Ala Gly Ala Gln Leu

                                     215                                    245
GTC GCA TTT CCC GAA TTG TGG ATT CCG GGC TAC CCA GGA TTC ATG CTC ACG CAC AAC CAA
Val Ala Phe Pro Glu Leu Trp Ile Pro Gly Try Pro Gly Phe Met Leu Thr His Asn Gln

                                     275                                    305
ACC GAA ACC CTA CCA TTC ATC ATT AAA TAC CGC AAG CAG GCA ATC GCC GCC GAT GGA CCA
Thr Glu Thr Leu Pro Phe Ile Ile Lys Try Arg Lys Gln Ala Ile Ala Ala Asp Gly Pro

                                     335                                    365
GAA ATC GAA AAA ATT CGC TGC GCG GCT CAG GAG CAT AAC ATT GCG CTC TCC TTT GGG TAC
Glu Ile Glu Lys Ile Arg Cys Ala Ala Gln Glu His Asn Ile Ala Leu Ser Phe Gly Tyr

                                     395                                    425
AGC GAA CGG GCT GGC CGT ACT CTC TAC ATG TCA CAA ATG CTT ATC GAT GCC GAT GGC ATC
Ser Glu Arg Ala Gly Arg Thr Leu Tyr Met Ser Gln Met Leu Ile Asp Ala Asp Gly Ile

                                     455                                    485
ACC AAA ATT CGT CGT CGA AAG CTC AAA CCA ACC CGC TTT GAA CGA GAA CTC TTT GGC GAA
Thr Lys Ile Arg Arg Arg Lys Leu Lys Pro Thr Arg Phe Glu Arg Glu Leu Phe Gly Glu

                                     515                                    545
GGT GAC GGA TCG GAC TTA CAG GTC GCC CAA ACT AGC GTT GGT CGG GTG GGT GCC CTC AAC
Gly Asp Gly Ser Asp Leu Gln Val Ala Gln Thr Ser Val Gly Arg Val Gly Ala Leu Asn

                                     575                                    605
TGC GCG GAG AAT TTG CAG TCG CTA AAC AAC TTT GCG CTT GCT GCC GAG GGT GAA CAG ATA
Cys Ala Glu Asn Leu Gln Ser Leu Asn Lys Phe Ala Leu Ala Ala Glu Gly Glu Gln Ile
```

```
                                         635                                         665
CAT ATC TCC GCC TGG CCA TTC ACG CTT GGA AGC CCT GTG CTC GTC GGA GAC TCC ATC GGC
His Ile Ser Ala Trp Pro Phe Thr Leu Gly Ser Pro Val Leu Val Gly Asp Ser Ile Gly

                                         695                                         725
GCC ATC AAC CAG GTC TAC GCG GCC GAG ACG GGG ACC TTC GTT CTC ATG TCG ACG CAG GTG
Ala Ile Asn Gln Val Tyr Ala Ala Glu Thr Gly Thr Phe Val Leu Met Ser Thr Gln Val

                                         755                                         785
GTT GGA CCG ACC GGC ATC GCC GCC TTC GAG ATC GAA GAC AGG TAC AAC CCG AAT CAG TAT
Val Gly Pro Thr Gly Ile Ala Ala Phe Glu Ile Glu Asp Arg Tyr Asn Pro Asn Gln Tyr

                                         815                                         845
CTT GGT GGT GGG TAC GCG CGG ATC TAC GGG CCT GAC ATG CAG TTG AAG AGC AAG TCG TTG
Leu Gly Gly Gly Tyr Ala Arg Ile Tyr Gly Pro Asp Met Gln Leu Lys Ser Lys Ser Leu

                                         875                                         905
TCA CCG ACC GAA GAG GGC ATC GTC TAC GCC GAG ATC GAC CTG TCG ATG CTT GAG GCA GCA
Ser Pro Thr Glu Glu Gly Ile Val Tyr Ala Glu Ile Asp Leu Ser Met Leu Glu Ala Ala

                                         935                                         965
AAG TAC TCG CTC GAT CCC ACG GGC CAC TAT TCG CGC CCT GAT GTG TTC AGC GTG TCG ATT
Lys Tyr Ser Leu Asp Pro Thr Gly His Tyr Ser Arg Pro Asp Val Phe Ser Val Ser Ile

                                         995                                        1025
AAC CGG CAA CGG CAG CCT GCG GTG TCA GAA GTT ATC GAC TCA AAC GGT GAC GAG GAC CCG
Asn Arg Gln Arg Gln His Ala Val Ser Glu Val Ile Asp Ser Asn Gly Asp Glu Asp Pro

                                        1055                                        1085
AGA GCA GCA TGC GAG CCC GAC GAG GGG GAT CGT GAG GTC GTA ATC TCT ACG GCA ATA GGG
Arg Ala Ala Cys Glu Pro Asp Glu Gly Asp Arg Glu Val Val Ile Ser Thr Ala Ile Gly

                                        1115                                        1155
GTT CTA CCC CGT TAT TGC GGA CAT TCC TAATAAAAAGAGACACGTGGTACCAAAGGGGTGTTCATGTCCA
Val Leu Pro Arg Tyr Cys Gly His Ser

                                        1200
GACGCAGAAAATATAGCCCAGAGTTAAAACGCGAAGCCATCGCTTTAACCCGTCAAC
```

The PstI-HincII fragment substantially free of 5'- and 3'-non-coding flanking regions may be ligated with EcoRI linkers, digested with EcoRI and is now ready to be introduced into a plant expression cassette by insertion into the EcoRI site of pCGN451.

pCGN451 includes an octopine cassette which contains about 1,566bp of the 5' non-coding region fused via an EcoRI linker to the 3' end of the gene and about 1,349bp of 3' non-coding DNA. The pTi coordinates are 11,207 to 12,823 for the 3' region and 13,643 to 15,208 at the 5' region as defined by Barker et al., Plant Molecular Biology (1983) 2:335. The 5' fragment was obtained as follows: A small subcloned fragment containing the 5' end of the coding region, as a BamHI-EcoRI fragment was cloned in pBR322 as plasmid pCGN407. The BamHI-EcoRI fragment has an XmnI site in the coding region, while pBR322 has two XmnI sites. pCGN407 was digested with XmnI, resected with Bal31 nuclease and EcoRI linkers added to the fragments. After EcoRI and BamHI digestion, the fragments were size fractionated, the fractions cloned and sequenced. In one case, the entire coding region and 10bp of the 5' non-translated sequences had been removed leaving the 5' non-transcribed region, the mRNA cap site and 16bp of the 5' non-translated region (to a BamHI site) intact. This small fragment was obtained by size fractionation on a 7% acrylamide gel and fragments approximately 130bp long eluted. This size fractionated DNA was ligated into M13mp9 and several clones sequenced and the sequence compared to the known sequence of the octopine synthase gene. The M13 construct was designated pI4, which plasmid was digested with BamHI and EcoRI to provide the small fragment which was ligated to an XhoI to BamHI fragment containing upstream 5' sequences from pTiA6 (Garfinkel and Nester, J. Bacteriol. (1980) 144:732) and to an EcoRI to XhoI fragment containing the 3' sequences.

The resulting XhoI fragment was cloned into the XhoI site of a pUC8 derivative, designated pCGN426. This plasmid differs from pUC8 by having the sole EcoRI site filled in with DNA polymerase I, and having lost the PstI and HindIII site by nuclease contamination of HincII restriction endonuclease, when a XhoI linker was inserted into the unique HincII site of pUC8. The resulting plasmid pCGN451 has a single EcoRI site for the insertion of protein coding sequences between the 5' non-coding region (which contains 1,550bp of 5' non-transcribed sequence including the right border of the T-DNA, the mRNA cap site and 16bp of 5' non-translated sequence) and the 3' region (which contains 267bp of the coding region, the stop codon, 196bp of 3' non-translated DNA, the polyA site and 1,153bp of 3' non-transcribed sequence).

The XhoI fragment containing the octopine synthetase (ocs) cassette was inserted into plasmid pCGN517, which has tetracycline resistance and kanamycin resistance genes. pCGN517 was prepared from pHC79 (Hohn, Gene (1980) 11:291) by introducing into the unique PstI site, the Kan$^r$ gene from pUC4K (Vieira, Gene (1982) 19:259). pCGN517 was digested with SalI and the XhoI fragment inserted into the unique SalI site.

The XhoI fragment was also inserted into a second plasmid pCGN529. pCGN529 is prepared from pACYC184 by insertion of the Kan$^r$ gene from Tn5 (Rothstein et al., 1981, in Movable Genetic Elements, p. 99, Cold Spring Harbor Laboratories, Cold Spring Harbor, NY) and a BglII fragment of 2.4kb from pRiA4 T-LDNA (White and Nester, J. Bacteriol. (1980) 144:710) inserted into the BamHI site remaining after substitution of the HindIII-BamHI fragment of pACYC184 with the Kan$^r$ gene of Tn5.

The XhoI fragment containing the ocs cassette into which the EcoRI nitrilase gene is inserted at the unique EcoRI of the ocs cassette is inserted into pCGN517 and pCGN529 to give two plasmids pN1 and pN2, respectively, which are used for introduction into A. tumefaciens or A. rhizogenes, respectively, for integration to the T-DNA of the Ti- or Ri-plasmids. Integration into the respective plasmids can be achieved in a 3-way mating as described by Comai et al., Plasmid (1983) 10:21-30. Overnight cultures of E. coli host containing plasmids pRK2073, pN1 or pN2 and A. tumefaciens A722 (Garfinkel, J. Bacteriol. (1980) 144:732) or A. rhizogenes A4T (White, ibid. (1980) 144:710) are cultured overnight and the appropriate cultures mixed and spread on AB plates containing 150µg/ml kanamycin. Single colonies are restreaked twice. Correct integration is verified by Southern analysis of total Agrobacterium DNA. Endonuclease digested DNA is probed with nick-translated pBrx8.

## The bromoxynil specific nitrilase gene is expressed in gall tissue.

The plasmid pBrx9, which carries the nitrilase gene on a 2.6 kb fragment, was digested with BamHl and treated with Bal31 to remove some 5' flanking region. BamHl linkers were added and reclosure was accomplished. The resulting plasmids which provided ampicillin resistance were transformed into E. coli as described previously and transformants selected on ampicillin selective medium to provide 5.2 kb plasmids pBrx16 and pBrx17, which carry the nitrilase gene on a 2.6 kb fragment. pBrx16 was digested with BamHl and partially digested with HincII resulting in the 1.2 kb nitrilase gene fragment.

The BamHl-HincII fragment was inserted into BamHl-SmaI digested pCGN46 to provide the 6.6kb plasmid pBrx22 containing the nitrilase gene fragment.

PCGN46 (Comai et al., Nature (1985) 317:741-744) is a mannopine synthase (MAS) expression cassette and contains a MAS promoter and ocs 3' region. Construction of pCGN46 was accomplished in the following manner. An approximately 5.5kbp EcoRI fragment (Ecol3 or EcoC) carrying a portion of the T-R DNA (Barker et al., Plant Mol. Biol. (1983) 2:325) including the mannopine synthase promoter region ($P_{MAS}$) was cloned in a vector designated pVK232. After digestion of pVK232 with EcoRI, Ecol3 was inserted into the EcoRI site of pACYC184 to yield plasmid pCGN14. pCGN14 was digested with SphI and ClaI (respectively at position 21562 and 20128 of the Barker et al. sequence, supra) to remove the $P_{MAS}$ region which was inserted into pUC19 (Pharmacia, Inc.) which had been digested with SphI and AccI to yield PCGN40. The $P_{MAS}$ region includes a ClaI recognition site internally which is methylated, so as to resist digestion.

pCGN40 was digested with EcoRV and EcoRI where the EcoRV site is in the T-DNA, while the EcoRI site is in the polylinker of pUC19 to provide a fragment having the $P_{MAS}$ region. pCGN451 containing the octopine synthase cassette was digested with SmaI and EcoRI and the larger fragment isolated from which the octopine synthase 5' region had been removed. The EcoRV-EcoRI $P_{MAS}$ region was substituted into pCGN451 for the octopine synthase 5' region, where the transcriptional initiation and termination regions were separated by a polylinker to provide pCGN46.

The plasmid pBrx22 containing the 1.2 kb nitrilase gene fragment was transformed into E. coli as described previously. The plasmid was isolated in conventional ways and digested with Xho I to provide a 4.1 kb fragment containing MAS promoter, bromoxynil gene containing 25 base pairs of bacterial 5' untranslated sequence and ocs 3' region. The 4.1 kb fragment was inserted into the SalI-digested plasmid pCGN783 to provide the approximately 31 kb plasmid pBrx28.

## Construction of pCGN783
## Construction of pCGN167

To construct pCGN167, the AluI fragment of CaMV (bp 7144-7735) (Gardner et al. Nucl. Acids Res. (1981) 9:2871-2888) was obtained by digestion with AluI and cloned into the HincII site of M13mp7 (Vieira Gene (1982) 19:259) to create C614. An EcoRI digest of C614 produced the EcoRI fragment from C614 containing the 35S promoter which was cloned into the EcoRI site of PUC8 (Vierra et al., Gene (1982) 19:259) to produce pCGN146.

To trim the promoter region, the BglII site (bp 7670) was treated with BglII and Bal31 and subsequently a BglII linker was attached to the Bal31 treated DNA to produce pCGN147.

pCGN148a containing a promoter region, selectable marker (KAN with 2 ATG's) and 3' region was prepared by digesting pCGN528 (see below) with BglII and inserting the BamHI-BglII promoter fragment from pCGN147. This fragment was cloned into the BglII site of pCGN528 so that the BglII site was proximal to the kanamycin gene of pCGN528.

The shuttle vector used for this construct, pCGN528, was made as follows. pCGN525 was made by digesting a plasmid containing Tn5 which harbors a kanamycin gene (Jorgenson et al. Mol. Gen. (1979) 177:65) with HindIII-BamHI and inserting the HindIII-BamHI fragment containing the kanamycin gene into the HindIII-BamHI sites in the tetracycline gene of pACYC184 (Chang & Cohen J. Bacteriol. (1978) 134,1141-1156). pCGN526 was made by inserting the

BamHI fragment 19 of pTiA6 (Thomashow et al. Cell (1980) 19:729-739) into the BamHI site of pCGN525. pCGN528 was obtained by deleting the small XhoI fragment from pCGN526 by digesting with XhoI and religating.

pCGN149a was made by cloning the BamHI kanamycin gene fragment from pMB9KanXXI into the BamHI site of pCGN148a.

pMB9KanXXI is a pUC4K variant (Vieira & Messing, Gene (1982) 19:259:268) which has the XhoI site missing but contains a functional kanamycin gene from Tn903 to allow for efficient selection in Agrobacterium.

pCGN149a was digested with BglII and SphI. This small BglII-SphI fragment of pCGN149a was replaced with the BamHI-SphI fragment from M1 (see below) isolated by digestion with BamHI and SphI. This produces pCGN167, a construct containing a full length CaMV promoter, 1ATG-kanamycin gene, 3' end and the bacterial Tn903-type kanamycin gene. M1 is an EcoRI fragment from pCGN550 (see construction of pCGN587) and was cloned into the EcoRI cloning site of M13mp9 in such a way that the PstI site in the 1ATG-kanamycin gene was proximal to the polylinker region of M13mp9.

Construction of 709 (1ATG-Kanamycin - 3' region)

pCGN566 contains the EcoRI-HindIII linker of pUC18 (Yanisch-Perron, ibid) inserted into the EcoRI-HindIII sites of pUC13-cm (K. Buckley, Ph.D. thesis, UC-San Diego, 1985). The HindIII-BglII fragment of pNW31c-8, 29-1 (Thomashow et al. (1980) Cell 19:729) containing ORF1 and 2 (Barker et al. (1983), supra) was subcloned into the HindIII-BamHI site of pCGN566 producing pCGN703.

The Sau3A fragment of pCGN703 containing the 3' region of transcript 7 from pTiA6 (corresponding to bases 2396-2920 of pT115955 (Barker et al. (1983), supra) was subcloned into the BamHI site of pUC18 (Yanisch-Perron et al. (1985), supra) producing pCGN709.

Construction of pCGN766c (35s promoter - 3' region)

The HindIII-BamHI fragment of pCGN167 (for construction see infra) containing the CaMV-35S promoter, 1ATG-kanamycin gene and the BamHI fragment 19 of pTiA6 was cloned into the BamHI-HindIII sites of pUC19 (Norrander et al. (1983), supra; Yanisch-Perron et al. (1985), supra) creating pCGN976.

The 35S promoter and 3' region from transcript 7 was developed by inserting a 0.7kb HindIII-EcoRI fragment of pCGN976 (35S promoter) and the 0.5kb EcoRI-SalI fragment of pCGN709 (transcript 7:3', for construction, see supra), into the HindIII-SalI sites of pCGN566 creating pCGN766c.

Final Construction of pCGN783

The 0.7kb HindIII-EcoRI fragment of pCGN766c (CaMV-35S promoter) was ligated to the 1.5kb EcoRI-SalI fragment of pCGN726c (1-ATG-KAN-3' region) into the HindIII-SalI sites of pUC119 (J. Vieira, Rutgers University, N.J.) to produce pCGN778.

The 2.2kb region of pCGN778, HindIII-SalI

fragment containing the CaMV 35S promoter (1-ATG-KAN-3' region) replaced the HindIII-SalI polylinker region of pCGN739 to produce pCGN783.

pBrx17 was digested with BamHl and partially digested with HincII resulting in the 1.2kb nitrilase gene fragment. The BamHl - HincII fragment was inserted into BamHl - SmaI digested pCGN566 to provide the 3.7kb plasmid pBrx25 containing the nitrilase gene fragment.

pCGN566 was constructed in the following manner. pUC13 (Cm$^R$) (Ken Buckley Ph.D. thesis, U.C., San Diego) was digested with EcoRI and HindIII and polylinkers from pUC18 and pUC19 were inserted respectively into the linearized pUC13 to give pCGN566 which carries a chloramphenicol resistance marker.

The plasmid pBrx25 containing the 1.2kb nitrilase gene fragment was transformed into E. coli as described previously. The plasmid was isolated in conventional ways and digested with BamHl and EcoRI to provide again the 1.2kb nitrilase gene fragment. The BamHl and EcoRI fragment was inserted into the BamHl and EcoRI digested pCGN46 to provide the 6.6kb plasmid pBrx27 containing the nitrilase gene fragment.

pBrx27 was transformed into E. coli as described previously. The plasmid was isolated in conventional ways and digested with XhoI to provide a 4.1kb fragment containing MAS promoter, bromoxynil gene containing 11 base pairs of bacterial 5' in translated sequence and ocs 3' region. The 4.1kb fragment was inserted into SAlI - digested pCGN783 to provide the approximately 31kb plasmid pBrx29.

### Detection of nitrilase expression

Plasmids pBrx28 and pBrx29 were transformed into the Agrobacterium tumefaciens strain K12. (Nester, Ann. Rev. Micro. (1981) 35: 531. Hoekema et al., Nature (1983) 303: 179) K12 (pBrx28) and K12 (pBrx29) were used to form galls on Kalanchöe (Garfinkel, J. Bacteriol. (1980) 144: 732).

About 1gm (fresh weight) of gall tissue was ground in liquid nitrogen in buffer containing 0.1M Tris pH 7.5, 10mM EDTA, 0.15M NaCl, 0.05% NP-40, 25 mg/ml BSA, 1mM DTT and 0.13 ug/ml leupeptin. Samples were homogenized after the addition of 0.05g polyvinylpyrrolidone (Sigma), then centrifuged at 15,000g for 15 min. at 4°C. 25 ul of antiserum, prepared by injecting purified nitrilase into rabbits, and 250ul 10% (w/v) suspension of S. aureus (Calbiochem) were added to each supernatant and incubated for 16 hr. at 4°C. Samples were then centrifuged and the pellet washed twice with 20mM Tris pH 7.5, 1mM EDTA, 150mM NaCl and 0.05% NP-40. The pellets were resuspended in 100ul 0.125M Tris pH 6.8, 4% SDS, 20% glycerol and 10% BMe and heated for 2 min. at 90°C. The entire sample was electrophoresed on 10% acrylamide gels (Laemmli, V.K. Nature 227: 680-685 (1970)). The resolved polypeptides were transferred to nitrocellulose filters (Schleicher and Schuell) as described by Burnette (Anal. Biochem. 112: 195-203 (1981)). Nitrocellulose filters (Schleicher & Schuell) were then incubated in BLOTTO (Johnson et al, Gen. Anal.

Technol. 1, 38-42 (1983)) for 1-3 hrs. at 42°C. followed by overnight incubation at room temperature in BLOTTO containing a 1:50 solution of anti-nitrilase serum. Filters were washed for 10 min. in 20mM Tris pH 7.5, 150mM NaCl; for 20 min. in the same buffer containing 0.05% Tween-20 and for another 10 min. in buffer without Tween-20. BLOTTO containing $10^6$ cpm/ml of $^{125}$I-labelled protein A (9u Ci/mg; NEN) was then added to filters and incubated at room temperature for 2 hrs. The filters were washed overnight in 50mM Tris pH 7.5, 1M NaCl and 0.4% Sarkosyl. After rinsing and drying, filters were exposed to Kodak AR X-ray film at -70°C. using a Dupont Cronex intensifying serum.

Transformation and regeneration of tobacco leaf slices co-cultivated with A. rhizogenes.

Tobacco plants are cultivated axenically (25°C, white light (16hr); MS (1mg/L IAA, 0.15mg/L kinetin). Three-week-old plants maintained through main shoot transplant are used as tissue donors. Young leaves (down to the fourth from the top) are selected, leaf disks 2mm in diameter are punched out and placed in Petri dishes (3cm in diameter) in 1ml of MS medium with 1mg/L IAA. After keeping the disks overnight in total darkness, Agrobacterium (A722xpN1 or pN2) cells ($10^8$-$10^9$/ml in plant culture medium) are added to these cultures. Co-cultivation is carried out for 18-24hr in darkness. Leaf slices are freed from Agrobacterium by washing 3x with MS medium lacking hormones and containing 350mg/L cefotaxine (Boehringer-Mannheim). Leaf slices are transferred in 9cm Petri dishes in 10ml of MS medium without hormones. Phytagar (Gibco, 0.6%; cefotaxine, 350mg/L) Petri dishes are sealed with parafilm and kept under the same conditions as tissue donor plants. Roots appear up to 2-4 weeks, are excised and placed under the same conditions in the same medium plus 2mg/L IAA and 2mg/L kinetin. Regenerating shoots are visible in the following 2-5 weeks.

Plants are sprayed at the 6-leaf stage by directing a spray of bromoxynil solution toward the potted plant. Each 4" pot contains a plant and receives 2.5ml of spray. Plants are grown in a growth chamber at 25°C, 70% relative humidity, 60hr light period. Growth is scored 9 days after spraying by counting the new leaves longer than 0.5cm.

By following the above procedures, plants cam be obtained which are bromoxynil resistant and can be used in the field in the presence of bromoxynil without significant adverse effect on their growth.

The subject invention provides for the improvement of plants by making them herbicidal resistant, particularly to specific benzonitrile herbicides.. Thus, the gene encoding for the nitrilase may be introduced into a plant host, whereby the gene will be expressed and impart benzonitrile resistance to the plant. In addition, the enzyme can be produced by cloning of the gene in a convenient bacterial host, whereby the enzyme is expressed. Enzymes having activity which can be monitored find a wide variety of uses, in assays for various analytes or for the benzonitrile substrate. In addition, the enzymes and bacteria expressing the enzymes can be used for removing the benzonitrile herbicide from contaminated environments.

CLAIMS

1. An expression cassette comprising a structural gene coding for a 3,5-dihalogenated-p-hydroxybenzonitrile specific nitrilase under the transcriptional and translational regulatory control of regulatory regions functional in a plant cell.

2. An expression cassette according to claim 1 wherein the 3,5-dihalogenated-p-hydroxybenzonitrile is 3,5-dibromo-p-hydroxybenzonitrile or 3,5-diiodo-p-hydroxybenzonitrile.

3. An expression cassette according to claim 1 or 2 wherein the nitrilase is a bacterial nitrilase.

4. An expression cassette according to claim 1, 2 or 3 wherein the cassette has at least one T-DNA border.

5. An expression cassette according to any one of claims 1 to 4 wherein the transcriptional initiation region is from a gene encoding an opine.

6. A vector which incorporates an expression cassette according to any one of claims 1 to 5.

7. A vector according to claim 6 which is a plasmid.

8. A plasmid capable of replication in E. coli or A. tumefaciens which plasmid comprises an expression cassette according to any one of claims 1 to 5.

9. A plasmid according to claim 8 wherein the expression cassette has at least one T-DNA border.

10. A bacterial host having a foreign gene capable of expressing a nitrilase specific for a 3,5-dihalogenated-p-hydroxybenzonitrile.

11. A bacterial host according to claim 10 wherein the nitrilase is specific for 3,5-dibromo- or 3,5-diiodo-4-hydroxybenzonitrile.

12. A bacterial host according to claim 10 or 11 which is E. coli.

13. A composition comprising a nitrilase having a molecular weight of about 34 kDal, the composition having a specific activity of at least about 0.1 $\mu$mol $NH_3$/min/mg protein with bromoxynil as substrate.

14. A composition according to claim 13 which is a bacterial nitrilase.

15. A composition according to claim 13 or 14 which is obtained from Klebsiella.

16. A composition according to claim 13, 14 or 15 which has a specific activity of at least about 0.5 $\mu$mol $NH_3$/min/mg protein.

17. A culture of Eschericia coli MM294 strain (pBrx5) in a medium comprising a source of assimilable carbon, a source of assimilable nitrogen and, optionally, mineral elements and substantially free of other microorganisms.

18. Eschericia coli MM294 strain (pBrx5).

19. A process for the preparation of a 3,5-dihalogenated-p-hydroxybenzonitrile specific nitrilase which comprises culturing a bacterial host according to claim 10, 11, 12 or 18.

20. A 3,5-dihalogenated-p-hydroxybenzonitrile specific nitrilase obtained by a process according to claim 19.

21. A DNA sequence encoding a 3,5-dihalogenated-p-hydroxybenzonitrile specific bacterial nitrilase and which is joined to and under the transcriptional regulatory control of other than the wild type transcriptional initiation region for a bromoxynil and/or ioxynil specific bacterial nitrilase found in Klebsiella.

22. A DNA sequence according to claim 21 wherein the transcriptional regulatory region is functional in a plant.

23. A DNA sequence according to claim 21 wherein the transcriptional regulatory region is functional in a bacterium.

24. A DNA sequence according to claim 21 substantially as hereinbefore defined in the Example.

25. A DNA sequence having an open reading frame coding for a nitrilase enzyme specific for a 3,5-dihalogenated-p-hydroxybenzonitrile, the sequence having at its 5'-terminus other than the wild type transcriptional initiation region.

26. A DNA sequence according to claim 25 wherein the 3,5-dihalogenated-p-hydroxybenzonitrile is 3,5-dibromo-p-hydroxybenzonitrile or 3,5-diiodo-p-hydroxybenzonitrile.

27. A DNA sequence according to claim 25 or 26 wherein the nitrilase enzyme is a bacterial nitrilase enzyme.

28. A DNA sequence according to claim 25 or 26 substantially homologous with a DNA sequence from Klebsiella.

29. A plant cell comprising an expression cassette according to any one of claims 1 to 5.

30. A plant comprising a plant cell according to claim 29.

31. A method for producing a nitrilase specific for a 3,5-dihalogenated-p-hydroxybenzonitrile which comprises:

isolating K. ozaenae which produce nitrilase specific for said 3,5-dihalogenated-p-hydroxybenzonitrile;

growing K. ozaenae in an appropriate nutrient medium; and

lysing said K. ozaenae and isolating said nitrilase.

32. A method for obtaining an enzyme having a selectable property which comprises:

screening bacteria for said selectable property and selecting said bacteria having said selectable property;

cleaving the genome of said bacteria to produce fragments of a desired size range;

cloning said fragments on appropriate vectors in a bacterium and selecting for enzymes having said selectable property; and

isolating the DNA sequence having the structural gene expressing said enzyme having said selectable property.